# EUROPÄISCHE PATENTANMELDUNG

(11) **EP 4 687 150 A1**
(43) Veröffentlichungstag der Anmeldung: **04.02.2026**
(21) Anmeldenummer: 25193386.7
(22) Anmeldetag: 01.08.2025
(51) Int. Cl.: G16H 20/40

(54) **VERFAHREN ZUR PRÜFUNG UND FREIGABE EINES VIRTUELLEN MODELLS**

(30) Priorität: 02.08.2024 DE 102024122172
(71) Anmelder: SCHEU-DENTAL GmbH, 58642 Iserlohn (DE)
(72) Erfinder: DUDAI, Daniela, 56283 Nörtershausen (DE); AICHINGER, Florian, 84036 Landshut (DE); SIVAK, Matias Gabriel, 40225 Düsseldorf (DE)
(74) Vertreter: Bauer PSU PartG mbB

(57) **Zusammenfassung**

Offenbart ist ein Verfahren zur Prüfung und Freigabe eines virtuellen, aus einer Anzahl von Dreiecken bestehenden Modells (1) für die Produktion eines Aligners, der Zähne eines Kiefers überdeckt und dem Abziehen von dem Kiefer in einer Raumrichtung (d) eine Retention entgegensetzt, wobei jedem der Dreiecke ein eineindeutiger Index zugewiesen und ein Flächeninhalt, eine aus dem Kiefer heraus weisende Oberflächennormale sowie ein Winkel zwischen der Oberflächennormalen und der Raumrichtung (d) und ein Kosinusbetrag des jeweiligen Winkels (αᵢ) berechnet wird, und der Aligner nur dann zur Produktion anhand des Modells (1) freigegeben wird, wenn die Retention größer als eine vorgegebene Minimalretention und kleiner als eine vorgegebene Grenzretention ist, und andernfalls das Modell (1) verworfen wird.

Um die Retention des Aligners anhand des Modells (1) zu beurteilen wird vorgeschlagen, dass zu jedem der Dreiecke eine Projektion zu 0 gesetzt wird, wenn der jeweilige Winkel kleiner als 90 ° ist, und andernfalls die Projektion als Produkt des jeweiligen Flächeninhalts mit dem Kosinusbetrag bestimmt wird, und die Retention als Summe der Projektionen bestimmt wird.

## Beschreibung

Die Erfindung betrifft ein Verfahren zur Prüfung und Freigabe eines virtuellen, aus einer Anzahl von Dreiecken bestehenden Modells für die Produktion eines Aligners, der Zähne eines Kiefers überdeckt und einem Abziehen von dem Kiefer in einer Raumrichtung eine Retention entgegensetzt, wobei jedem der Dreiecke ein eineindeutiger Index zugewiesen und ein Flächeninhalt, eine aus dem Kiefer heraus weisende Oberflächennormale sowie ein Winkel zwischen der Oberflächennormalen und der Raumrichtung und ein Kosinusbetrag des jeweiligen Winkels berechnet wird, und der Aligner nur dann zur Produktion anhand des Modells freigegeben wird, wenn die Retention größer als eine vorgegebene Minimalretention und kleiner als eine vorgegebene Grenzretention ist, und andernfalls das Modell verworfen wird. Die Erfindung betrifft weiter ein Verfahren zum Konfigurieren eines solchen Aligners.

Aligner sind ohne Werkzeug in den Kiefer einsetz- und aus diesem herausnehmbare zahnmedizinische Geräte, die aus einer dünnen, meist transparenten Kunststofffolie zur Korrektur von Zahnfehlstellungen individuell für den jeweiligen Patienten angefertigt werden. Im Verlauf der zahnmedizinischen Behandlung werden Aligner regelmäßig ausgetauscht, um die Zähne schrittweise bis zum Ende in die jeweils gewünschte Position zu bringen.

In dem aus US 2023/0,325,558 A1 bekannten Verfahren wird ein virtuelles Modell des Aligners auf Grundlage eines dreidimensionalen Scans des Kiefers derart konstruiert und anhand des Modells der Aligner gefertigt, dass die Folie an der Oberfläche der Zähne anliegt. Unterschnitte an den Zähnen werden hierbei genutzt, um auf einzelne Zähne Zugkräfte aus dem Aligner in Richtung zur Kaufläche (okklusal) bzw. zur Schneidekante (inzisal) zu erzeugen. Diese Zugkräfte dürfen sowohl in Summe als auch an jedem einzelnen Zahn einen Grenzwert nicht überschreiten, damit sie dem regelmäßigen Entnehmen des Aligners keinen übergroßen Widerstand - die sogenannte "Retention" - entgegensetzen. In dem bekannten Verfahren wird die Retention als Anzahl oder als Summe der Winkel über 90 ° bestimmt.

### Aufgabe

Der Erfindung liegt die Aufgabe zugrunde, die Retention besser zu bestimmen.

### Lösung

Ausgehend von den bekannten Verfahren wird nach der Erfindung vorgeschlagen, dass automatisch zu jedem der Dreiecke eine Projektion zu 0 gesetzt wird, wenn der jeweilige Winkel kleiner als 90 ° ist, und andernfalls die Projektion als Produkt des jeweiligen Flächeninhalts mit dem Kosinusbetrag bestimmt wird, und die Retention als Summe der Projektionen bestimmt wird.

Die Zerlegung einer Oberfläche eines dreidimensionalen Modells in Dreiecke (sog. "Triangulation") als Grundlage der computergestützten Bearbeitung des Modells ist ebenso allgemein bekannt wie die Berechnung einer Oberflächennormalen eines Dreiecks und eines Winkels zwischen zwei Richtungen im Raum - hier: der Raumrichtung zum Abziehen und der jeweiligen Oberflächennormalen.

Dreiecke, deren Oberflächennormale einen Winkel kleiner als 90 ° zur Raumrichtung zum Abziehen aufweist, sind zu der Raumrichtung geneigt und setzen dem Abziehen des Aligners keinen Widerstand entgegen, tragen also zur Retention des Aligners in der Raumrichtung nicht bei. Ist der Winkel größer als dieser Wert, dann ist das jeweilige Dreieck gegen die Raumrichtung geneigt und werden zur Bestimmung der Retention berücksichtigt. Bereiche im Kiefer, in denen solche Dreiecke vorliegen, werden als Unterschnitte, Undercut oder "unter sich gehende Bereiche" bezeichnet. In erster Näherung ist der Beitrag eines Dreiecks in einem Unterschnitt zur Retention proportional zum Flächeninhalt einer Projektion des Dreiecks in der Raumrichtung, genauer: in eine Ebene senkrecht zu der Raumrichtung, in der der Aligner abgezogen wird.

Der Flächeninhalt dieser Projektion entspricht dem Produkt des Flächeninhalts des jeweiligen Dreiecks mit dem Kosinusbetrag (dem Betrag des Kosinus) des Winkels zwischen der Raumrichtung und der Oberflächennormalen des Dreiecks. Die Summe der Flächeninhalte dieser Projektionen ist ein Maß zur Beschreibung des Widerstands der betrachteten Oberfläche des Aligners gegen das Abziehen in der Raumrichtung.

Ein bestehendes Modell eines Aligners wird nach dem erfindungsgemäßen Verfahren automatisch anhand einfacher Zahlenwerte - der Minimal- und der Grenzretention - auf seine Eignung zum Abziehen in der gewählten Raumrichtung geprüft und zur Produktion des entsprechenden Aligners freigegeben oder verworfen.

Vorzugsweise wird in einem erfindungsgemäßen Verfahren jede der Projektionen vor der Summierung mit dem Kosinusbetrag des jeweiligen Winkels multipliziert. Unter sich gehende Bereiche des Kiefers tragen umso stärker zur Retention bei, je stärker sie gegen die Raumrichtung geneigt sind, in der der Aligner abgezogen wird. In erster Näherung entspricht die Abhängigkeit von der Neigung wiederum dem Kosinusbetrag des Winkels der Oberflächennormalen des jeweiligen Dreiecks zu der Raumrichtung. Die Quadrierung erspart in einem solchen erfindungsgemäßen Verfahren die Berechnung des Betrags des Kosinus.

Vorzugsweise wird in einem erfindungsgemäßen Verfahren vor dem Vergleich mit der Grenzretention eine Gesamtfläche des in der Raumrichtung projizierten Aligners bestimmt und die Retention durch die Gesamtfläche dividiert. Die projizierte Gesamtfläche kann in einem erfindungsgemäßen Verfahren bestimmt werden, indem die Alignerkante in eine Ebene orthogonal zur Abzugsrichtung projiziert und der Flächeninhalt des resultierenden Polygons berechnet wird. In einem solchen erfindungsgemäßen Verfahren ist die Retention als Maß des Widerstands gegen das Abziehen eine dimensionslose Größe. Die Normierung auf die projizierte Gesamtfläche des Aligners erlaubt den Vergleich unterschiedlicher Aligner und die Definition einer allgemein gültigen - gleichfalls dimensionslosen - Grenzretention.

Vorzugsweise deckt in einem solchen erfindungsgemäßen Verfahren das Modell nur einzelne der Zähne ab. In einem solchen erfindungsgemäßen Verfahren wird nur eine Teilretention des Aligners bezogen auf die jeweils von dem Modell abgedeckten Zähne geprüft. Weiter vorzugsweise wird das erfindungsgemäße Verfahren sowohl für einzelne Zähne als auch für den Aligner als Ganzes durchgeführt, wobei Grenzretention für einzelne Zähne oder Zahngruppen und für den Aligner als Ganzes getrennt bestimmt werden.

Vorzugsweise wird ein virtuelles Modell eines Aligners, der Zähne eines Kiefers überdeckt, automatisch iterativ konfiguriert, indem in dem Modell mindestens einer der Zähne mit einem Add-on versehen und/oder in einem Unterschnitt ausgeblockt wird, der Aligner gemäß einem vorgenannten Verfahren geprüft wird, und eine Raumrichtung ermittelt wird, in der die Retention minimal ist, bis die Add-ons eine gewünschte Zahnbewegung erzielen und der Aligner freigegeben ist. Insbesondere kann die Wahl der Raumrichtung, der Positionierung von Add-ons und zum Ausblocken für einen Schritt der Iteration automatisch durch eine künstliche Intelligenz erfolgen.

Vorzugsweise werden in einem solchen erfindungsgemäßen Verfahren die Add-ons automatisch an eine Krümmung des jeweiligen Zahns angepasst. In einem erfindungsgemäßen Verfahren liegen die Oberfläche des Kiefers und die Add-ons in triangulierter Form vor. Lokale Abstände zwischen Zahn und Add-on lassen sich so mit bekannten Verfahren leicht ermitteln und das Add-on entsprechend anpassen.

Vorzugsweise umfassen in einem solchen erfindungsgemäßen Verfahren die Add-ons aktive und/oder passive Attachments, Elastics, Power Ridges, Press Points, Bite Ramps, Bite Blocks und/oder Cut-Outs. Attachments sind kleine Kunststoffteile, die auf die Zähne geklebt werden, um dem Aligner zusätzliche Haltepunkte zu geben. Sie helfen, kompliziertere Zahnbewegungen zu ermöglichen, wie Drehungen oder Verschiebungen in vertikaler Richtung. Elastics (Gummibänder) werden verwendet, um den Druck auf die Zähne zu erhöhen und bestimmte Bewegungen zu unterstützen, wie das Schließen von Lücken oder das Korrigieren von Überbissen. Sie werden an speziellen Haken an den Alignern befestigt. Power Ridges sind kleine Rillen oder Erhöhungen auf den Alignern, die dazu dienen, gezielten Wurzeltorque zu erleichtern. Bite Ramps werden an den Alignern angebracht, um den Biss zu öffnen und so Bewegungen der hinteren Zähne zu erleichtern. Sie werden häufig bei der Behandlung von tiefen Bissen verwendet. Bite Blocks (auch: Bite-Turbo oder okklusale Rampe) sind kleine, in der Regel aus Komposit bestehende Aufsätze, die während der kieferorthopädischen Behandlung, einschließlich der Aligner-Therapie, auf die Zähne gesetzt werden. Sie erfüllen mehrere wichtige Funktionen, um die Wirksamkeit und den Komfort der Behandlung zu verbessern.

Die Retention wird weiterhin durch die Folienstärke und durch die Abschnittskurve (Elshazly et al., Effect of trimming line design..., Journal of Dentistry 125 (2022) 104276 und Journal of Mechanical Behaviour of Biomedical Materials 140 (2023) 105741) beeinflusst. Auch diese Parameter können in einem erfindungsgemäßen Verfahren automatisch variiert werden.

In einem erfindungsgemäßen Verfahren nutzt die Automatik vorzugsweise eine KI.

### Ausführungsbeispiel

Die Erfindung wird nachfolgend anhand eines Ausführungsbeispiels erläutert. Es zeigen
- Fig. 1: ein virtuelles Modell für einen Aligner,
- Fig. 2: eine Formel für die erfindungsgemäße Prüfung,
- Fig. 3: einen Zahn mit vorkonfektionierte Add-Ons und
- Fig. 4: den Zahn mit individuell angepassten Add-Ons.

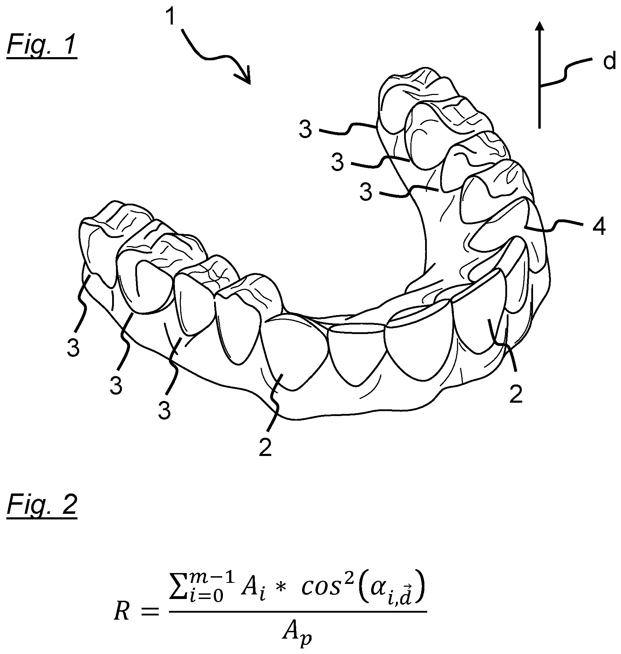

In einem erfindungsgemäßen Verfahren wird geprüft, ob das in Figur 1 gezeigte virtuelle 3D-Modell 1 zur Produktion eines entsprechenden Aligners mit einer vorgegebenen Raumrichtung d zum Abziehen vom Kiefer geeignet ist. In dem Modell 1 sind Add-Ons 2 angebracht und Unterschnitte 3 ausgeblockt.

Im Modell 1 ist die zur Außenseite des Modells 1 gerichtete Oberfläche der Dreiecke jeweils durch den Umlaufsinn der Knoten definiert.

Zu jedem der Dreiecke wird ein Flächeninhalt Aᵢ, eine von der Außenseite des Modells 1 ausgehende Oberflächennormale nᵢ, ein Winkel αᵢ zwischen der

Oberflächennormalen nᵢ und der Raumrichtung d und eine Projektion als Produkt des Flächeninhalts Aᵢ mit dem Kosinusbetrag des Winkels αᵢ berechnet.

Für jedes Modell wird automatisch eine individuelle Raumrichtung d zum Abziehen des Aligners von dem Kiefer bestimmt, für die die Retention R minimal wird.

Die Alignerkante wird in eine Ebene orthogonal zur Raumrichtung d projiziert und die Gesamtfläche Aₚ des Aligners als Flächeninhalt des resultierenden Polygons berechnet.

Die Projektionen der Dreiecke, die einen Winkel αᵢ größer als 90 ° aufweisen, werden erneut mit dem Kosinusbetrag gewichtet und dann summiert, die Summe wird als Maß der Retention R auf die Gesamtfläche Aₚ normiert.

Geprüft wird, ob die Retention R des Aligners als Ganzes und an den von dem Aligner überdeckten einzelnen Zähnen des Kiefers beim Abziehen in der Raumrichtung d eine vorgegebenen Grenzretention Aₘₐₓ übersteigt.

Der Aligner wird zur Produktion anhand des Modells 1 freigegeben, wenn die Retention R kleiner als die Grenzretention Aₘₐₓ ist, andernfalls wird das Modell 1 verworfen. In letzterem Fall werden Add-Ons 2 und Ausblockung mittels einer künstlichen Intelligenz im Modell 1 modifiziert und die Eignung des modifizierten Modells 1 erneut wie oben geprüft:

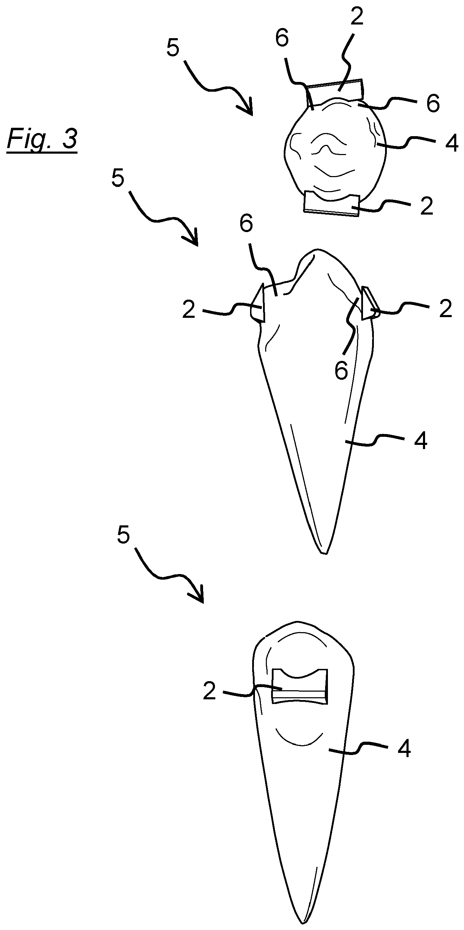

Figur 3 zeigt in drei Ansichten einen Zahn 5 des Kiefers mit vorläufig positionierten Add-Ons 2. Die Add-Ons 2 sind aktive Attachments (sog. "Bite Ramps"), die als vorgefertigte Elemente in verschiedenen Ausführungen zu Verfügung stehen. Aufgrund der natürlichen Krümmung der Oberfläche 4 des Zahns 5 stehen die vorgefertigten Add-Ons 2 nur punktförmig mit diesem in Kontakt. Beim Anbringen müssen die Zwischenräume 6 zwischen den Add-Ons 2 und der Oberfläche 4 manuell mit Kleber aufgefüllt werden. Die Position der Add-Ons 2 auf der Oberfläche 4 kann deshalb in der Praxis signifikant von der geplanten Stellung abweichen.

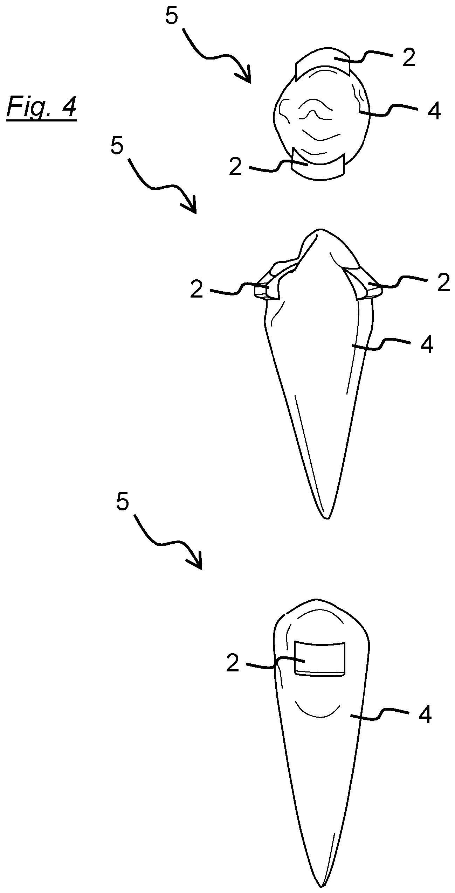

Im Rahmen eines erfindungsgemäßen Verfahrens werden vorgefertigte Add-Ons 2 zunächst derart positioniert, dass ein mittlerer Abstand zu den Dreiecken in der Oberfläche 4 des Zahns 5 minimal ist. In dieser Position überschneiden sich die Volumina des Zahns 5 und des jeweiligen Add-Ons 2. Figur 4 zeigt Zahn 5 mit im Rahmen eines erfindungsgemäßen Verfahrens an die Oberfläche 4 individuell angepassten Add-Ons 2. Die individualisiert hergestellten Add-Ons 2 schmiegen sich genau in der vorgesehenen Position ohne Zwischenraum eng an die Oberfläche 4 des Zahns 5 an. Die der Planung entsprechende Positionierung ist so deutlich erleichtert.

In den Figuren sind
- 1: Modell
- d: Raumrichtung
- 2: Add-On
- 3: Unterschnitt
- 4: Oberfläche
- Aᵢ: Flächeninhalt
- αᵢ: Winkel
- Aₚ: Gesamtfläche
- R: Retention
- 5: Zahn
- 6: Zwischenraum

## Patentansprüche

1. Verfahren zur Prüfung und Freigabe eines virtuellen, aus einer Anzahl (n) von Dreiecken bestehenden Modells (1) für die Produktion eines Aligners, der Zähne eines Kiefers überdeckt und dem Abziehen von dem Kiefer in einer Raumrichtung (d) eine Retention (R) entgegensetzt, wobei jedem der Dreiecke ein eineindeutiger Index (i) zugewiesen und ein Flächeninhalt (Aᵢ), eine aus dem Kiefer heraus weisende Oberflächennormale (nᵢ) sowie ein Winkel (αᵢ) zwischen der Oberflächennormalen (nᵢ) und der Raumrichtung (d) und ein Kosinusbetrag des jeweiligen Winkels (αᵢ) berechnet wird, und der Aligner nur dann zur Produktion anhand des Modells (1) freigegeben wird, wenn die Retention (R) größer als eine vorgegebene Minimalretention (Rₘᵢₙ) und kleiner als eine vorgegebene Grenzretention (Rₘₐₓ) ist, und andernfalls das Modell (1) verworfen wird. ***dadurch gekennzeichnet, dass*** automatisch zu jedem der Dreiecke eine Projektion zu 0 gesetzt wird, wenn der jeweilige Winkel (αᵢ) kleiner als 90 ° ist, und andernfalls die Projektion als Produkt des jeweiligen Flächeninhalts (Aᵢ) mit dem Kosinusbetrag bestimmt wird, und die Retention (R) als Summe der Projektionen bestimmt wird.

2. Verfahren nach dem vorgenannten Anspruch, ***dadurch gekennzeichnet, dass*** jede der Projektionen vor der Summierung mit dem Kosinusbetrag des jeweiligen Winkels (αᵢ) multipliziert wird.

3. Verfahren nach einem der vorgenannten Ansprüche, ***dadurch gekennzeichnet, dass*** vor dem Vergleich mit der Grenzretention (Rₘₐₓ) eine Gesamtfläche (Aₚ) des in der Raumrichtung (d) projizierten Aligners bestimmt und die Retention (R) durch die Gesamtfläche (Aₚ) dividiert wird.

4. Verfahren nach dem vorgenannten Anspruch, ***dadurch gekennzeichnet, dass*** das Modell (1) nur einzelne der Zähne abdeckt.

5. Verfahren zum Konfigurieren eines virtuellen Modells (1) eines Aligners, der Zähne eines Kiefers überdeckt, ***dadurch gekennzeichnet, dass*** automatisch iterativ in dem Modell (1)
• mindestens einer der Zähne mit einem Add-on versehen und/oder in einem Unterschnitt (3) ausgeblockt wird,
• der Aligner gemäß einem der Ansprüche 1 bis 4 geprüft wird, und
• eine Raumrichtung (d) ermittelt wird, in der die Retention (R) minimal ist, bis die Add-Ons (2) eine gewünschte Zahnbewegung erzielen und der Aligner freigegeben ist.

6. Verfahren nach dem vorgenannten Anspruch, ***dadurch gekennzeichnet, dass*** die Add-Ons (2) automatisch an eine Krümmung des jeweiligen Zahns (5) angepasst werden.

7. Verfahren nach einem der Ansprüche 5 und 6, ***dadurch gekennzeichnet, dass*** die Add-Ons (2) aktive und/oder passive Attachments, Elastics, Power Ridges, Press Points, Bite Ramps, Bite Blocks und/oder Cut-Outs umfassen.

8. Verfahren nach einem der Ansprüche 5 bis 7, ***dadurch gekennzeichnet, dass**,* die Automatik eine KI nutzt.
